# EUROPEAN PATENT APPLICATION

(11) **EP 4 047 351 A1**
(43) Date of publication of application: **24.08.2022**
(21) Application number: 21157707.7
(22) Date of filing: 17.02.2021
(51) Int. Cl.: G01N 21/31, G01N 21/3563, G01N 21/359, G01N 21/85, G01N 33/02, G01N 33/10

(54) **METHOD FOR DETECTING PLANT DISEASES AND PHYSIOLOGICAL PROPERTIES INFLUENCEABLE BY ENVIRONMENTAL STRESS IN PLANT MATERIAL**

(71) Applicant: KWS SAAT SE & Co. KGaA, 37574 Einbeck (DE)
(72) Inventor: Hilscher, Elke, 37574 Einbeck (DE); Narten, Heiko, 30966 Hemmingen (DE); Meldau, Stefan, 37077 Göttingen (DE)

(57) **Abstract**

The invention relates to a method for detecting plant diseases in plant material and/or physiological properties influenceable by environmental stress in plant material, an analysis assembly for detecting plant diseases in plant material, an arrangement for detecting plant diseases in plant material, and a control unit for controlling an analysis assembly and/or for receiving data from an analysis assembly.

## Description

The invention relates to a method for detecting plant diseases in plant material and/or physiological properties influenceable by environmental stress in plant material, an analysis assembly for detecting plant diseases in plant material, an arrangement for detecting plant diseases in plant material, and a control unit for controlling an analysis assembly and/or for receiving data from an analysis assembly.

In breeding, field trail, and/or in plant processing industries, such as the sugar industry, plant diseases can occur in plant material used therein. The infection of plant material with plant diseases is typically detrimental, as plant diseases can dramatically change the properties of the plant material. Many biotic agents, such as virus, fungi, bacteria, nematodes, insects, molluscs, mammals, arthropods can cause imbalance in the physiology of the vital plant material parts. These biotic agents can attack the tissue of the plant material and cause chemical changes in the systemic tissues that are not affected. For example, maize leaf infection by fungi can cause physiological changes in the root of the plant material, even if the roots of the plant material are not infected (Balmer et al, The Plant Journal (2013) 74, 213-225). In addition, leaf infestation by pests can cause root metabolite changes in tea plants (Yang et al. J. Agric. Food Chem. 2019, 67, 19, 5465-5476). Furthermore, different leaf pests affect root metabolic responses in cabbage plants (Karssemeijer et al, Plant Cell Environ. 2020; 43:775-786.). Sugar beet root and crown rot, induced by the soil-borne fungus Rhizoctonia solani, has an influence on quality components in sugarbeets. *R. solani* infestation led to an enormous increase of reducing sugars as glucose and fructose. The molassegenic components sodium and amino-N showed minimal or no reaction to infestation, whereas potassium increased with increasing disease severity (J. Buddemeyer; B. Märländer; Journal of Plant Diseases and Protection Vol. 112, No. 2; 105-117; 2005). These examples show that chemical changes can be measured throughout the plant material after infection of local tissues. Both parasite and non-parasites diseases cause a great deal of damage and are characterized by wilting, scabs, mold coatings, rusts, and blotches, holes and rotted tissue.

At the whole plant level all stress conditions as drought stress, heat stress, salt stress are usually perceived as a decreases in photosynthesis and growth. Plants have evolved several adaptive mechanisms that allow the photochemical and biochemical system to cope with negative changes in environment. (I. Yordanov; V. Velikova; T. Tsonev; Plant response to drought, acclimation, and stress tolerance; Photosyntetica 38(1); 171-186; 2000).

Response of Sugar beet genotypes to drought and nutrient deficiency stress included leaf RWC (relative water content), glycine betaine accumulation, alteration of shoot/root ratio and production off fibrous roots (B. Shaw, T.H. Thomas; D.T. Cooke; Responses of sugar beet (Beta vulgaris L.) to drought and nutrient deficiency stress; Plant Growth Regulation (37) 77-83, 2002)

Determining plant diseases in plant material is relatively time consuming as well as labour and cost intensive. Furthermore, typically only a small portion of the plant material is analysed to determine if plant diseases are present in the plant material. However, if only parts of the plant material are infected with plant diseases, such plant infections can be overlooked, resulting in unreliability of the detection of plant diseases.

It is an object of the present invention to provide an improved method for detecting plant diseases in plant material, and/or to provide an improved analysis assembly for detecting plant diseases in plant material, and/or to provide an improved arrangement for detecting plant diseases in plant material, and/or to provide an improved control unit for controlling an analysis assembly and/or for receiving data from an analysis assembly. In particular, it is an object of the present invention to provide a solution for reliable and automated detection of plant diseases in plant material.

According to a first aspect, it is provided a method for detecting plant diseases in plant material and/or physiological properties influenceable by environmental stress in plant material, the method comprising receiving plant material, emitting electromagnetic waves towards the plant material, receiving electromagnetic waves, converting the received electromagnetic waves into a spectral signal, and preferably processing the spectral signal, determining plant diseases in the plant material and/or physiological properties, in particular metabolites, influenceable by environmental stress in the plant material.

The "plant material" that may be used in the present invention encompasses various embodiments of plant, including but not limited to, the leaf, root, beet, tuber, stem, fruit, and other plant tissues of any section of the plant, callus or an adventive embryo-like tissue (referred to hereinafter as "callus, etc." or simply "callus"), or a complete plant, A preferable form of the plant material used in the method of the present invention is root, beet or tuber.

Plant material can be root crops and/or tuber crops, in particular of the species Beta vulgaris and/or Solanum tuberosum. Plant material can be a part of root crops and/or tuber crops, in particular of the species *Beta vulgaris* and/or *Solanum tuberosum.* Preferably, plant material comprises or is one or several of the following group: sugar beet, spinach beet, swiss chard, beetroot, mangelwurzel, potato. Plant material can be a small or large part of a plant material portion, in particular a part of a plant material portion that is used for production processes and/or breeding processes. Plant material can comprise or consist of one or several parts of a plant. Plant material can for example comprise or consist of a leaf or a part thereof, in particular a stamped-out part of a leaf. Plant material can for example comprise or consist of several leaves or parts thereof, in particular a stamped-out parts of several leaves. Preferably, receiving plant material is conducted by receiving a truckload of plant material and/or a container of plant material and/or a goods wagon of plant material and/or an amount of plant material from a plot for breeding processes and/or an amount of plant material for field trail and/or plant material of a green house.

Preferably, the electromagnetic waves that are emitted the plant material lie within the infrared spectrum (0,7 µm - 1000µm) and/or in the microwave region (2 cm⁻¹ - 130 cm⁻¹) and/or in the visible spectrum (380 nm - 750 nm) and/or in the ultraviolet spectrum. (10 nm - 380 nm)

Preferably, the spectral signal is converted by using spectroscopy, in particular near-infrared spectroscopy (NIRS) mid-infrared spectroscopy and/or far-infrared spectroscopy and/or terahertz spectroscopy and/or ultraviolet-visible spectroscopy (UV-Vis) and/or Raman spectroscopy and/or laser-induced breakdown spectroscopy (LIBS) and/or fluorescent spectroscopy, and/or images as well as hyperspectral images and/or combination of images and/or hyperspectral images with spectroscopy methods and/or combinations of different spectroscopic methods and/or fluorescence imaging.

Preferably, the electromagnetic waves are reflected from the plant material and the reflected electromagnetic waves are received, in particular with a sensor that is adapted to receive electromagnetic waves. Preferably, the electromagnetic waves are reflected from the plant material, or a part thereof. The electromagnetic waves can also be, at least partly, emitted through the plant material and the electromagnetic waves can be received, in particular with a sensor that is adapted to receive electromagnetic waves. Preferably, the electromagnetic waves are emitted through the plant material, or a part thereof.

Preferably, the received electromagnetic waves are converted into a spectral signal, wherein the spectral signal is generated dependent on the received electromagnetic waves.

Preferably, the method comprises processing the spectral signal for determining plant diseases in the plant material. Processing the spectral signal for determining plant diseases in the plant material can be carried out to determine plant diseases in the plant material or at least in a part of the plant material. Preferably, plant diseases are determined dependent on the spectral signal, in particular dependent on information of the spectral signal.

Plant diseases can for example be determined in sugar beets. Preferably, determining plant diseases comprises determining if one or several of the following plant diseases, in particular sugar beet diseases, are present in the plant material and/or determining an infestation rate of one or several of the following plant diseases, in particular sugar beet diseases:
- Rhizomania,
- Beet soil-borne virus infection,
- Beet mild yellowing virus infection,
- Aphanomyces root rot,
- Rhizoctonia root and crown rot,
- Violet root rot,
- Phymatotrichum root rot,
- Phytophthora root rot,
- Pythium root rot,
- Phoma root rot,
- Beet vascular necrosis and rot,
- Crown gall,
- Scab,
- Syndrome basses richesse,

Pest infestation, caused by one or several of the following group: wireworm, cutworm, moth, aphid, pygmy mangold beetle, silver Y moth, spotted snake millipede, beet weevil, tortoise beetle, flea beetle, cicade, moth species, cockchafer, crane fly, springtail, darkling beetle, beet fly, beet leafminer, beet moth, garden or glass house symphylid, springtail, southern sugar beet weevil, true weevil, Lixus subtilis Sturm, Lygus, bright-line brown-eye, African cotton, leafworm, grey beet weevil, two-spotted spider mite, thrips.

Preferably, determining plant diseases and pest infestations comprises determining if one or several of the plant diseases and pest infestations shown in the following table, in particular sugar beet diseases, are present in the plant material and/or induced a change in the plant material and/or determining an infestation rate of one or several of the following plant diseases, in particular sugar beet diseases:

| **Bacteria and Rickettsia** | |
|---|---|
| **Common name of disease** | **Causal organisms** |
| Bacterial vascular necrosis and rot | *Pectobacterium carotovorum* supsp. *betavasculorum* |
| Bacterial leaf spot | *Pseudomonas syringae* p.v. *aptata* |
| Yellow wilt | *Phytoplasma* (rickettsia-like organism) |
| Syndrome des Basses Richesses (SBR) | Stolbur phytoplasma and proteobacterium |
| Beet latent rosette | Rickettsia-like organism |
| Beet scab | *Streptomyces scabies* |
| Soft rot | *Pectobacterium carotovora* supsp. *carotovora* |
| Bacterial pocket | *Pantoea agglomerans* p.v. *betae* |
| Silvering disease | *Curtobacterium flaccumfaciens* p.v. *betae* |
| Crown gall | *Agrobacterium tumefaciens* |

| **Fungi and Oomycetes** | **Causal organisms** |
|---|---|
| Cercospora leaf spot | *Cercospora beticola* |
| Ramularia leaf spot | *Ramularia beticola* |
| Phoma leaf spot | *Phoma betae* |
| Alternaria blight | *Alternaria alternata* and *A. brassicae* |
| Rhizoctonia foliar blight | *Rhizcotonia solani* |
| Powdery mildew | *Erysiphe polygoni (syn E. betae)* |
| Downy mildew | *Peronospora farinosa* f. sp. *betae* |
| Beet tumor / Crown wart | *Physoderma leproides* |
| Beet rust | *Uromyces betae* |
| Gray mold / Botrytis blight | *Botrytis cinerea* |
| Aphanomyces seedling disease | *Aphanomyces cochlioides* |
| Rhizoctonia seedling disease | *Rhizoctonia solani* |
| Pythium seedling disease | *Pythium ultimum* and *P. aphanidermatum* |
| Phoma seedling disease | *Phoma betae* |
| Aphanomyces chronic root rot | *Aphanomyces cochlioides* |
| Charcoal rot | *Macrophomina phaseolina* |
| Fusarium yellows | *Fusarium oxysporum* f. sp. *betae* |
| Fusarium root rot | *Fusarium oxysporum* f. sp. *radicis-betae* |
| Phoma root rot | *Phoma betae* |
| Phytophthora root rot | *Phytophthora cryptogea* and *P. drechsleri* |
| Rhizoctonia root and crown rot | *Rhizcotonia solani* |
| Violet root rot | *Helicobasidium brebissonii* |
| Rhizopus root rot | *Rhizopus stolonifer* and *R. oryzae* |
| Phymatotrichum root rot | *Phymatotrichum omnivorum* |
| Sclerotium root rot | *Sclerotium rolfsii* |
| Verticillium wilt | *Verticillium albo-atrum* and *V. dahliae* |
| | |

| **Viruses** | **Causal organisms** |
|---|---|
| Rhizomania | Beet necrotic yellow vein virus (BNYVV) |
| Beet soilborne mosaic virus | Beet soilborne mosaic virus (BSBMV) |
| Beet soilborne virus | Beet soilborne virus (BSBV) |
| Beet virus Q | Beet virus Q (BVQ) |
| Beet yellows virus | Beet yellows virus (BYV) |
| Beet western yellows virus | Beet western yellows virus (BWYV) |
| Beet chlorosis virus | Beet chlorosis virus (BChV) |
| Beet mild yellowing virus | Beet mild yellowing virus (BMYV) |
| Beet yellow stunt | Beet yellow stunt (BYSV) |
| Beet curly top | Beet curly top virus (BCTV) |
| Cucumber mosaic virus | Cucumber mosaic virus (CMV) |
| Beet mosaic | Beet mosaic virus (BtMV) |
| Sugar beet leaf curl | Beet leaf curl virus (BLCV) |
| Lettuce infectious yellows | Lettuce infectious yellows virus (LIYV) |
| Beet yellow blotch | Tobaco rattle virus (TRV) |
| Beet yellow net disease | Beet yellow net virus (BYNV) |
| | |

| **Nematodes** | **Causal organisms** |
|---|---|
| Sugar beet cyst nematode | *Heterodera schachtii* |
| Root-knot nematodes | *Meloidogyne* spp. |
| False root-knot nematodes | *Nacobbus spp.* |
| Stem and bulb nematode | *Ditylenchus dipsaci* |
| Clover / yellow cyst nematode | *Heterodera trifolii* |
| Stubby-root nematodes | *Trichodorus* spp. |
| Needle nematodes | *Longidorus* spp. |
| | |

| **Insects and arthropod pests** | **Causal organisms** |
|---|---|
| Sugar beet root maggot | *Tetanops myopaeformis* |
| Palestriped flea beetle | *Systena blanda* |
| Beet carrion beetle | *Aclypea spp.* |
| Beet flea beetles | *Chaetocnema* spp., *Phyllotetra* spp., *Epitrix* spp. |
| Tortoise beetle | *Cassida spp.* |
| Wireworms | *Agriotes* spp. |
| White grubs | *Phyllophaga* spp. |
| Cockchafer / chafer grubs | *Melolontha* spp. |
| Symphylids | *Scutigurella immaculata* |
| Millipedes | *Blaniulus gutulatus* |
| marsh cranefly / leatherjacket | *Tipula paludosa* |
| Pigmy mangold beetle | *Atomaria linearis* |
| Green peach aphid | *Myzus persicae* |
| Black bean aphid | *Aphis fabae* |
| Sugar beet root aphid | *Pemphigus betae* |
| Slugs | *Deroceras reticulatum, D. agreste* |
| Bugs | *Piesma quadrata* |
| Lygus bugs | *Lygus* spp., *Calocoris* spp., *Dolycoris* spp., *Mesocerus* spp. |
| Springtails | *Onychiurus armatus, O. fimatus, O. campatus* |
| Sugar beet crown borer | *Hulstia undulatella* |
| Beet petiole borer | *Cosmobaris americana* |
| Webworms | *Loxostege* spp. |
| Army cutworm | *Euxoa auxiliaris* |
| Pale western cutworm | *Agrotis orthogonia* |
| Beet armyworm | *Spodoptera exigua* |
| Fall armyworm | *Spodoptera frugiperda* |
| Beet moth | *Scrobipalpa ocellatella* |
| Silver Y moth | *Autographa gamma* |
| Cabbage moth | *Mamestra brassicae* |
| Springtails | *Bourletiella hortensis* |
| Grasshoppers | *Melaoplus* spp. |
| Leaf-feeding weevil | *Tanymecus confusus* and *T. palliatus* |
| Beet root weevil | *Bothynoderes punctiventris* |
| Beet weevil | *Conorhynchus medicus* |
| Beet lixus | *Lixus juncii* |
| Beet leaf miner | *Pegomya betae* and *P. hyoscyami* |
| Spider mites | *Tetranychus urticae* |
| Beet leaf hopper | *Circulifer tenellus* |
| Yellow leaf hopper | *Paratanus exitiosus* |
| Whitefly | *Bemisia tabaci* |

Plant diseases can for example be determined in potatoes. Preferably, determining if one or several of the following plant diseases, in particular potato diseases, are present in the plant material, and/or determining an infestation rate of one or several of the following plant diseases, in particular potato diseases:
- Black dot (Colletotrichum coccodes),
- Black scurf *(Rhizoctonia solani)* and/or stem canker that can be observed on the stem of the plant material and/or the leaf roll of the plant material,
- Common scab (Streptomyces scabies),
- Corky ring spot (Tobacco rattle virus [TRV]), in particular spread by soilborne nematodes as a vector,
- Dry rot and skin stain symptoms (Fusarium spp.),
- Early blight (Alternaria solani and Alternaria alternata),
- Gangrene (Phoma exigua var. foveta),
- Late blight (Phytophthora infestans),
- Leaf roll (Potato Leaf Roll Virus [PLRV]), wherein in particular this virus also renders the plant more susceptible to Rhizoctonia solani,
- Leaf drop streak, rugose mosaic (Potato Virus Y [PVY]),
- Mop Top (Potato Mop Top Virus [PMTV]),
- Pink eye (unknown causal agent, thought to be bacterial),
- Pink Rot (Phytophthora erythroseptica),
- Powdery scab (Spongospora subterranea),
- Pythium Leak (Pythium spp.),
- Ring rot (Clavibacter michiganensis subsp. sepedonicus),
- Silver scurf (Helminthosporium solani),
- Skin spot (Polyscytalum pustulans),
- Soft rot (Erwinia carotovora subsp. atroseptica), wherein in particular this pathogen can cause blackleg, which is observed in the stem and leaves of the plant material,
- Spraing (Tobacco Rattle Virus [TRV]),
- Wart disease (Synchytrium endobioticum),
- Zebra chip (Candidatus Liberibacter solanacearum),
- Root knot nematode (Meloidogyne spp. [nematode]),
- Root lesion nematode (Pratylenchus penetrans [nematode]),
- Potato cyst nematode (Globodera spp. [nematode]),
- Potato rot nematode (Ditylenchus destructor [nematode]),
- Tuberworm (Phthorimaea operculella [moth larvae]),
- Wireworm (Limonius spp., Agriotes spp. and Ctenicera spp. [beetle larvae]).

Physiological changes in root crops due to growing or storage condition as well as stress can be also detected according to the present invention. Such changes include in particular:
- Black heart, in particular caused by lack of oxygen during storage which can cause the tissue to die from the inside out and turn black,
- Brown center, wherein in particular an area of dead pith cells can turn brown and can be caused by very wet conditions,
- Elephant hide, wherein in particular rough or thick skin can be present and can be caused by high temperatures, soil fertility, soil moisture, and/or chemical treatments,
- Greening, wherein in particular tubers turning green due to exposure to light,
- Growth cracks, wherein in particular cracks in the skin can be caused by environmental stress, nutritional imbalance, disease, and/ or herbicide injury,
- Hollow heart, wherin in particular a star- or lens-shaped hollow area in the centre of the tuber can be caused by uneven amounts of moisture during tuber development or a combination of other environmental factors,
- Jelly/sugar end, wherein in particular physiological condition can be caused by high temperatures and/or moisture stress during tuber development, which can result in pointed and shrivelled stem end of the tuber.

Physiological properties influenceable by environmental stress in plant material is preferably understood to be physiological properties changeable by and/or affectable by environmental stress in plant material.

Preferably, physiological properties influenceable by environmental stress in plant material is understood to be physiological properties changed by and/or affected by environmental stress in plant material. Physiological properties changed by and/or affected by environmental stress in plant material are in particular understood to be physiological properties of the plant material, wherein the physiological properties have been changed and/or affected by environmental stress.

Physiological properties influenceable by environmental stress in plant material can in particular be changed by one or several influences of the following list:
- abiotic stress conditions, such as drought, salinity, heat, cold, frost, UV, wind, soil hardiness, nutrient limitations or overdose (e.g. N, K, P, Na, Mg, ect. Macro- and Micro-nutrients),
- pH value of the soil,
- soil contamination, for example with heavy metals,
- Beneficial organisms: Biologicals,
- Herbicide-induced changes (e.g. Conviso, Glyphosate ect.)
- Agrochemical-induced changes (Fungicides, pesticides, Insecticides, adjuvants)
- Agricultural practices induced changes (damage from non-chemical weed control; damage from agricultural machinery, harvesting damages (e.g. topping),
- storage damages,
- presence of plants in specific developmental conditions (e.g. bolters).

The method steps can be carried out in an order that deviates from the listed order. However, it is preferred if the method steps are carried out in the listed order, in particular one after the other.

The solution described herein has the advantage that spectroscopy has the potential to predict plant biochemical constituents such as nutrients and secondary metabolite, indicative of biotic stress, such as plant diseases. Therefore, using spectroscopy, plant diseases can reliably be determined and/or detected in an automated manner. Spectroscopy is particularly advantageous, as infected plant material can have a different spectral information than healthy plant material. The symptoms and stress caused by the pathogens as well as by insects and/or environmental stress factors can change pigmentation, alter surface temperature, and/or change in metabolites or metabolites contents. For example, sugar beets can be susceptible to a number of different viruses that can be transmitted by insects, fungi, nematodes, seed and/or physical contact. All these viruses may decrease the potential yield of the root crop and for example can affect the extractability of sugar within a sugar production process.

Furthermore, the solution described herein has the advantage that some plant diseases can only or more accurately be determined and/or detected in specific parts of the plant material and with the solution described herein it is possible to analyse specific parts of the plant material, such as for example leaves that are part of the plant material.

Furthermore, the solution described herein has the advantage that from determining diseases and/or physiological properties influenceable by environmental stress further important information can be derived, for example on storage loss of components in the plant material. For example, sugar beets with plant diseases can have a higher loss in sugar content and/or a bad storability behaviour compared to sugar beets without plant diseases. Furthermore, plant diseases can have an influence on the consistency of the plant material. This can lead to problems during chopping and/or slicing of plant material. By gaining such further information, further steps, such as steps in a production processes, agronomic practices or breeding processes can be improved.

In addition, it is an advantage of the method described herein that actions can be undertaken based on the information obtained, including for example:
- optimizing growth conditions,
- choosing the right varieties for the local conditions,
- improving agricultural practices,
- improving storage conditions,
- improving breeding based on such conditions,
- adjusting processes in a production factory,
- improving production factory conditions,
- improving growing conditions.

According to a preferred embodiment, the method comprises generating calibration data, including taking a sample of plant material, preferably with and/or without plant diseases and/or with and/or without physiological properties influenceable by environmental stress, and preferably moving the sample along a sensor that is adapted to receive electromagnetic waves, calibration analysis of the plant material by evaluating plant diseases in the plant material, for example by visual scoring of a plant disease and/or by analysis of metabolites and/or components present in the plant material, spectroscopic analysis of the sample by, preferably continuously, emitting electromagnetic waves towards the sample, receiving electromagnetic waves, and converting the received electromagnetic waves into a spectral signal, comparing the results of the spectroscopic analysis with the results of the calibration analysis.

The sample of plant material can for example comprise of 10 kg or 20 kg or 30 kg of plant material. Other amounts of plant material can also be used. Preferably, receiving electromagnetic waves can be understood as receiving electromagnetic waves that are reflected from and/or transmitted through and/or emitted by the sample. Calibration analysis can comprise at least one or several measurements, such as for example visual scoring, wherein the at least one measurement can be conducted on the plant material. Preferably, by comparing the results obtained from the spectroscopic analysis with the results obtained from the calibration analysis, calibration data can be generated, Visual scoring of plant disease can also be conducted on plant material such as crops in the fields before sampling, in particular for leaf infection by Cercospora.

Preferably the spectroscopic analysis is carried out continuously. Preferably, calibration data is generated by repeatedly taking samples and conducting spectroscopic analyses of the samples. Preferably, calibration data is generated by repeatedly comparing results of spectroscopic analyses with results from calibration analyses. It is preferred if this procedure is repeated several times, in particular more than 100 times, preferably more than 1000 times. Preferably, this procedure conducted on plant material with different plant diseases and/or on plant material with different plant material conditions, such as on relatively wet plant material, on relatively dry plant material, on plant material with varying components and/or ingredients.

Generating calibration data can also be carried out in a static process, wherein the sample of plant material is not moved along a sensor. Preferably, within such a static process, the sample is located at a defined position, in particular under and/or over a sensor that is adapted to receive electromagnetic waves.

An advantage of generating calibration data in the described way is that the calibration data can be used for reliably determining plant diseases in plant material even for plant material with different components and/or properties and/or with different conditions.

Preferably, generating calibration data comprises developing and/or optimising and/or validating a calibration model, wherein developing and/or optimising and/or validating the calibration model comprises generating a calibration set, a cross-validation set, and an independent validation set. Preferably, the calibration set comprises results obtained from spectroscopic analysis of a first part of the sample of plant material. Preferably, the cross-validation set comprises results obtained from spectroscopic analysis of a second part of the sample of plant material. Preferably, the independent validation set comprises results obtained from spectroscopic analysis of a third part of the sample of plant material. It is preferred if the first part, the second part, and the third part of the sample are parts of different and independent samples of plant material. The calibration model can be generated from the calibration set. The cross-validation set can be used for developing, in particular improving, the calibration model. The independent validation set can be used for validating the calibration model. It is preferably that the independent validation set is not used for changing and/or developing the calibration model. However, it is also possible to use examples from the independent validation set for extending or improving calibration.

According to a preferred embodiment, the method comprises comparing the spectral signal with calibration data and/or at least one reference value and dependent on the comparison determine presence and/or quantification and/or qualification of plant diseases and/or physiological properties influenceable by environmental stress in the plant material.

Preferably, a specific value, for example at a specific wavelength of a spectral signal can be compared with a reference value. Dependent on the characteristic reflectance spectrum based on the stress imposed on the plant (e.g. diseases) a comparison of the specific value with the reference value, it can be determined if plant diseases and/or physiological properties influenceable by environmental stress are present in the plant material. The spectral signal can be compared with calibration data and dependent on the comparison of the calibration data and the spectral signal, it can be determined if plant diseases and/or physiological properties influenceable by environmental stress are present in the plant material. The said reference value is understood by a person skilled in the art, e.g. a value generated based on qualitative and/or quantitative component analysis and/or scoring analysis and/or rating numbers and/or climatic data of samples of plant material. The qualitative and quantitative method include visual rating, detection by imaging system. For example, image analysis technologies can pave the way for an agile systems biology approach to create reference value, also the combination of feature selection and growth modelling supporting the biological interpretation of plant growth, physiological changes, and stress tolerance.

It is further preferred, if the comparison of the spectral signal with the calibration data and/or the at least one reference value can be used to determine the presence of plant diseases and/or physiological properties influenceable by environmental stress in the plant material. It is further preferred, if the comparison of the spectral signal with the calibration data and/or the at least one reference value can be used to determine the quantification of plant diseases and/or physiological properties influenceable by environmental stress in the plant material, in particular the number of plant diseases and/or the intensity of the one or more plant diseases present in the plant material and/or the intensity of environmental factors. It is further preferred, if the comparison of the spectral signal with the calibration data and/or the at least one reference value can be used to conduct a qualification of plant diseases or environmental factors in the plant material, in particular by determining a severity and/or a class of the plant diseases or class of environmental stress factors present in the plant material.

It is particularly advantageous to use calibration data and/or reference values for this comparison, as in such a way, plant diseases and/or environmental influences can be determined in an automated and reliable way.

According to a further preferred embodiment, generating calibration data and/or generating at least one reference value comprises conducting a plurality of reference spectral signals, wherein preferably the plurality of reference spectral signals is converted from electromagnetic waves reflected from and/or transmitted through plant material without plant diseases and/or plant material with plant diseases and/or plant material from different environmental regions, or at least a part thereof.

Preferably, the calibration data is derived using multiple and/or multivariate and/or linear regression analysis, and/or wherein the calibration data is derived from multivariate analysis including spectral preprocessing preferably by differentiating the spectral signals using mathematical classification options and/or filtering, in particular using mathematical filtering methods, and/or averaging spectral signals to one spectral signal, and/or carrying out multiple and/or multivariate and/or linear regression analysis for generating calibration data, wherein preferably the calibration data is derived using principle component analysis (PCA), and/or multiple linear regression (MLR), and/or partial least squares (PLS) regression, and/or machine learning, in particular using neuronal networks.

Preferably, the spectral signal is classified into a classification of infestation classes and/or different plant diseases and/or metabolites. In such a way, it is possible to classify plant diseases and environmental stress factors that are present in the plant material from information in the spectral signal.

According to a preferred embodiment of the method, determining plant diseases and/or physiological properties influenceable by environmental stress in the plant material comprises processing the spectral signal for determining plant diseases and/or physiological properties influenceable by environmental stress in the plant material, and/or wherein processing the spectral signal and/or generating calibration data comprises one or several of the following steps: preprocessing the spectral signal for correcting and/or eliminating overlaying effects, wherein preferably preprocessing is conducted using Standard Normal Variate (SNV) and/or multiplicative scatter correction (MSC), and/or first derivatives, and/or second derivatives, and/or smoothing and/or a combination of Derivative and SNV, wherein preferably preprocessing is conducted before multiple and/or multivariate and/or linear regression analysis is carried out, and/or removing spectral signals that are not converted from electromagnetic waves reflected from and/or emitted through the plant material, preferably by differentiating the spectral signals using classification and/or filtering, in particular using mathematical filtering methods and/or averaging spectral signals to one spectral signal.

According to a more preferred embodiment of the method, determining plant diseases and/or physiological properties influenceable by environmental stress in the plant material comprises processing the spectral signal for determining plant diseases and/or physiological properties influenceable by environmental stress in the plant material, and/or
wherein processing the spectral signal and/or generating calibration data comprises one or several of the following steps:
- preprocessing the spectral signal for correcting and/or eliminating overlaying effects, wherein preferably preprocessing is conducted using Standard Normal Variate (SNV) and/or multiplicative scatter correction (MSC), and/or first derivatives, and/or second derivatives, and/or smoothing, and/or combination of Derivative and SNV, wherein preferably preprocessing is conducted before multiple and/or multivariate and/or linear regression analysis is carried out, and/or
- and/or Classification as a learning method that classifies data into one of numerous already defined definite classes. It requires pre-allocation of training data in order to classify unknown data, thus classification is a so-called supervised learning method. Relevant algorithms for supervised learning are K-nearest Neighbor (kNN), Decision Trees/Random Forest, Support Vector Machines (SVM), Discriminant Analysis (PLS-DA), Soft independent modelling by class analogy (SIMCA), Naive Bayes (NB) as well as classifiers based on neural network and machine learning and/or
- Clustering as an unsupervised technique and suited to explore unknown data. Clustering algorithms map or group the input data into clusters according to similarities between them. Such grouping occurs by density-based, hierarchical-based, partitioning or grid-based methods but can also be achieved by machine learning or deep learning approaches. Common algorithms are K-means, mean shift, Gaussian Mixture, hidden Markov model (HMM) and Density-Based Spatial Clustering of Applications with Noise (DBSCAN).
- removing spectral signals that are not converted from electromagnetic waves that are reflected from or emitted through the plant material, preferably by differentiating the spectral signals using classification and/or filtering, in particular using mathematical filtering methods, and/or
- averaging spectral signals to one spectral signal.

An advantage of such a preprocessing is that the spectral signal can be corrected and failures in recorded spectral signals do not lead to poor or wrong results. If unwanted overlaying effects are present in the recorded spectral signals, these effects can be eliminated by using such a preprocessing.

Multiplicative scatter correction (MSC) is a standard normalization technique that aims to correct spectral signals in such a way that they are as close as possible to a reference spectrum, generally the mean of the data set, by changing the scale and the offset of the spectral signals.

Calculating the first derivatives and/or the second derivatives can also be used for preprocessing the obtained spectral signals.

Regression analysis is a set of statistical processes for estimating the relationships between a dependent variable, which is often called the 'outcome variable', and one or more independent variables, which is often called 'predictors', 'covariates', or 'features'. The most common form of regression analysis is linear regression, in which a line or a more complex linear combination is determined, wherein the line most closely fits the data according to a specific mathematical criterion.

Averaging spectral signals to one spectral signal can be conducted by averaging several spectral signals obtained during generating calibration data to one spectral signal that shows an average of the several spectral signals.

According to a further preferred embodiment, the method comprises: detecting metabolites and/or components of the plant material and/or determining sugar beet diseases in the plant material. A metabolite is in particular understood to be an intermediate or end product of metabolism.

According to a further preferred embodiment, the method comprises chopping and/or slicing the plant material into plant material pieces, and/or homogeneously distributing the plant material onto a transport device, preferably with a roller that is arranged above the transport device, and/or adjusting the plant material, in particular by adjusting the height of the plant material and/or by compressing the plant material and/or by smoothing the surface of the plant material, preferably with the roller.

Preferably, chopping the plant material into plant material pieces is conducted by cutting and/or crumbling the plant material into plant material pieces. After chopping the plant material into plant material pieces, the plant material pieces can still be referred to as plant material. Preferably, slicing the plant material into plant material pieces is conducted by slicing and/or cutting the plant material into plant material pieces. After slicing the plant material pieces, the plant material pieces can still be referred to as plant material.

Preferably conveying the plant material using the transport device is conducted along a transport direction, wherein a stream of the plant material is generated. Preferably, the stream of the plant material moves along the transport direction.

Preferably, the transport device is understood to be a device for conveying plant material. The transport device can be adapted to convey the plant material. Preferably, the transport device comprises a conveyor belt.

According to a further preferred embodiment, the method comprises arranging the, preferably homogeneously distributed, plant material onto a transport device, and/or conveying the plant material using the transport device, wherein preferably the plant material is conveyed towards a sensor, wherein the sensor is adapted to receive electromagnetic waves.

Preferably, the electromagnetic waves that are emitted towards the plant material lie within the infrared spectrum, in particular in the near-infrared spectrum, and/or in the visible spectrum and/or in the ultraviolet spectrum. Preferably, the electromagnetic waves have at least one wavelength, wherein the wavelength of the electromagnetic waves lies in the range of 10 nm to 3,000,000 nm, in particular in the range of 780 nm to 2500 nm, 10 preferably in the range of 850 nm to 1650 nm. Preferably, the spectral signal is converted by using spectroscopy, in particular near infrared spectroscopy and/or mid-infrared spectroscopy and/or far infrared-spectroscopy and/or terahertz spectroscopy and/or microwave-spectroscopy and/or ultraviolet-visible spectroscopy (UV-Vis) and/or Raman spectroscopy and/or laser-induced breakdown spectroscopy (LIBS), and or hyperspectral images, and/or fluorescence images and/or combination of hyperspectral images with spectroscopy methods and/or combinations of different spectroscopic methods.

Preferably, the electromagnetic waves are reflected from and/or emitted through the plant material that is arranged on the transport device. Preferably, the electromagnetic waves are received, in particular with a sensor that is adapted to receive electromagnetic waves. Preferably, the electromagnetic waves are reflected from and/or emitted through the plant material. Preferably, the received electromagnetic waves are converted into a spectral signal, wherein the spectral signal is generated dependent on the received electromagnetic waves.

According to a further preferred embodiment, the method comprises continuously recording received electromagnetic waves, and/or continuously converting the received electromagnetic waves into spectral signals.

Continuously recording received electromagnetic waves is particularly advantageous, as in such a way the method described herein can be applied onto continuous processes, such as for example continuous processes within a sugar production factory in which sugar beet pieces are continuously conveyed on a conveyor belt.

Continuously converting the received electromagnetic waves into spectral signals is particularly advantageous, as in such a way the method described herein can be applied onto continuous processes, such as for example continuous processes within a sugar production factory in which sugar beet pieces are continuously conveyed on a conveyor belt.

According to a preferred embodiment of the method, while emitting electromagnetic waves towards the plant material the plant material is arranged on the transport device and/or conveyed using the transport device, and/or wherein the wavelength of the electromagnetic waves lies in the ultraviolet (UV) spectrum and/or in the visible spectrum and/or in the infrared spectrum and/or in the microwave spectrum and/or in the terahertz-spectrum, wherein preferably the wavelength of the electromagnetic waves lies in the range of 10 nm to 3000 µm.

According to a preferred embodiment of the method, the electromagnetic waves are received from at least one spectrometer and/or at least one spectrophotometer.

Preferably, the at least one spectrometer and/or the at least one spectrophotometer are preferably of the following group: ultraviolet-visible (UV/Vis) spectrophotometer, near-infrared (NIR) spectrometer, Raman spectrometer. Near infrared spectroscopy and/or mid- infrared spectroscopy and/or far infrared spectroscopy and/or terahertz spectroscopy and/or microwave spectroscopy and/or ultraviolet-visible spectroscopy (UV-Vis) and/or Raman spectroscopy and/or laser-induced breakdown spectroscopy (LIBS), and or hyperspectral images and/or combination of hyperspectral images with spectroscopy methods and/or combinations of different spectroscopic methods

According to a preferred embodiment of the method, chopping the plant material into plant material pieces is conducted using a chopping device, wherein the chopping device is configured to cut and/or crumble the plant material into substantially equal sized plant material pieces and/or wherein slicing the plant material into plant material pieces is conducted using a slicing device, wherein the slicing device is configured to cut and/or slice the plant material into plant material pieces that are preferably formed as thin elongated strips and/or slices.

The plant material can be cut and/or crumbled with the chopping device and/or cut and/or sliced with the slicing device, wherein the chopping device and/or the slicing device comprises blades and/or knifes for cutting the plant material into plant material pieces. The plant material pieces can for example be formed as thin elongated strips, which are also often, and in particular in the sugar production industry, referred to as cossettes.

According to a preferred embodiment of the method, wherein the species of the plant material is a root crop and/or a tuber crop, in particular Beta vulgaris and/or Solanum tuberosum, or at least a part thereof, and/or wherein the plant material comprises or consists of sugar beets, and/or wherein the plant material is introduced to a production process for processing the plant material in a processing industry, and/or wherein the plant material is introduced to a breeding process.

The processing industry can for example be sugar production industry, potato processing industry or other plant processing industries. Breeding process is understood to be a plant breeding process, in particular for breeding the plant material.

According to a further preferred embodiment, the method comprises introducing plant material into a breeding process, and/or changing at least one breeding parameter, dependent on the determination of plant diseases in the plant material and/or physiological properties influenceable by environmental stress in the plant material. The breeding process could be changed e.g. by using results from the analysis with the method as described herein to select a plant that has increased resistance and/or tolerance to a particular stress condition. The breeding process could be changed e.g. by starting or strengthening a breeding program or weakening the input into a breeding program based on the information about the presence of stress conditions (e.g. diseases) revealed by the data gained from the spectroscopic analysis, e.g. if stress conditions are present in a specific number of economically important markets then it makes sense to invest more into the breeding. The breeding process could be changed e.g. by combining variety information (e.g. the genetic and/or genomic sequence of the varieties) with information about the detection of stress conditions (e.g. diseases), e.g. the data show that specific varieties/genotypes, with specific genomic sequences, have higher or lower levels of stress conditions (e.g. disease scoring based on the calibration) and the information is then used to select varieties/genotypes with the specific genomic sequences and introduce these specific genomic parts into other varieties/genotypes that lack these genomic parts.

Based on the data received from the spectroscopic analysis, the growth/cultivation conditions of the plants (field or glasshouse) could be improved in a way that reduces the stress condition for the plant. E.g.
- identifying locations that are prone to specific stress conditions (e.g. disease hotspots) and improving the yield or quality of the plants grown in these locations by selecting or recommending the right varieties that are adapted/tolerant/resistant for the stress conditions,
- improving agricultural practices in these locations (e.g. using agrochemicals such as pesticides/fungicides/biologicals to minimize disease pressure, specific soil preparations to reduce stress, watering of plants, applying nutrients, using specific farm equipment/technologies to reduce the stress condition, training of farmers, etc.),
- improving storage conditions,
- improving of transport conditions,
- improving seed preparations (e.g. adjusting seed coating with fungicide to increase fungal resistance),
- adjusting crop rotation.

Based on the data received from the spectroscopic analysis and combined with the geographic location and other environmental information it can be possible to improve the carbon footprint (i.e., less CO2 emissions) of a product (e.g. sugar). This information may be revealed to the consumer and therefore increases the price for the plant/crop product. Based on the data received from the spectroscopic analysis and combined with the geographic location and other environmental information it can be possible to identify the cultivation conditions and increase the traceability of such information throughout the supply chain. This information may be revealed to the consumer and therefore increases the price for the plant/crop product.

The data received from the spectroscopic analysis and combined with the geographic location and other environmental information might be of value to certain stakeholders in the agricultural industry and/or food industry and/or insurance industry and/or data industry and/or political decision makers and/or legal entities and or any other stakeholders. Such information could be made available or be implemented by digital farming platforms and/or combined with other data to create additional value. This method could improve marketing and sales for seed companies or agricultural suppliers by selecting or recommending the right varieties that are adapted/tolerant/resistant for the stress conditions.

According to a further preferred embodiment, the method comprises producing a product, preferably raw juice and/or sugar and/or starch, from the plant material, changing at least one sugar production parameter, in particular extraction parameters, and/or dosages of additives, and/or electric field pulses, and/or pulse numbers, and/or process temperature, and/or conveying speed and/or duration of plant material pieces in reactor and/or at least one drying process parameter, dependent on the determination of plant diseases and/or physiological properties influenceable by environmental stress in the plant material.

Preferably, raw juice is a product within a sugar production process, wherein sugar is produced from sugar beets. It is preferred to change at least one sugar production parameter dependent on the determination of plant diseases in the plant material, in particular because in such a way the sugar production process and in particular the sugar extraction can be improved. For example, the sugar extraction process for extracting sugar from sugar beet pieces can be adapted dependent on the determination of plant diseases in the sugar beets. Thus, the sugar extraction process can be adapted to the determined plant diseases that are currently in the sugar production process, which can for example lead to an improved sugar yield.

According to a further aspect, it is provided a method for generating calibration data for the determination of diseases in plant material and/or physiological properties influenceable by environmental stress in plant material, comprising taking a sample of plant material, preferably with and/or without plant diseases and/or with and/or without physiological properties influenceable by environmental stress, and preferably moving the sample along a sensor that is adapted to receive electromagnetic waves, calibration analysis of the plant material by evaluating plant diseases and/or physiological properties, in particular metabolites, influenceable by environmental stress in the plant material, for example by visual scoring and/or analysis of components and/or metabolism of a plant disease and/or physiological properties, in particular metabolites, influenceable by environmental stress present in the plant material, spectroscopic analysis of the sample by, preferably continuously, emitting electromagnetic waves towards the sample, receiving electromagnetic waves, and converting the received electromagnetic waves into a spectral signal, comparing the results of the spectroscopic analysis with the results of the calibration analysis.

According to a further aspect, it is provided an analysis assembly for detecting plant diseases in plant material and/or physiological properties influenceable by environmental stress in plant material, wherein the analysis assembly is arranged to emit electromagnetic waves towards plant material pieces, wherein the analysis assembly is arranged to receive reflected and/or emitted electromagnetic waves, wherein the analysis assembly is arranged to convert the received reflected electromagnetic waves into a spectral signal, wherein the analysis assembly is processing the spectral signal for determining plant diseases and/or physiological properties, in particular metabolites, influenceable by environmental stress in the plant material.

According to a further aspect, it is provided an arrangement for detecting plant diseases in plant material and/or physiological properties influenceable by environmental stress in plant material, the system comprising a receiving section for receiving plant material, a chopping device for chopping the plant material into plant material pieces, an analysis assembly described herein.

The receiving section can be a section and/or station for receiving plant material from transport vehicles, such as for example trucks and/or trains. The receiving section can be an inlet of the chopping device and/or slicing device, wherein the plant material is introduced to the chopping device and/or slicing through the inlet.

According to a further aspect, it is provided a control unit for controlling an analysis assembly as described herein and/or for receiving data from an analysis assembly as described herein and controlling at least one production parameter, in particular extraction parameters and/or dosages of additives, and/or electric field pulses and/or pulse numbers and/or process temperature and/or conveying speed and/or duration of plant material pieces in a reactor and/or at least one drying process parameter dependent on the determination of plant diseases in the plant material and/or physiological properties influenceable by environmental stress in the plant material.

The further aspects described above and their respective possible embodiments comprise features and/or method steps that are particularly suitable to be used with and/or connected with the method and its preferred embodiments described herein.

For the advantages, preferred embodiments, and details of the individual different aspects and their preferred embodiments, reference is also made to the description, and in particular to the described advantages, preferred embodiments, and details described with reference to the respective other aspects.

Further advantageous embodiments result from the combination of individual, several or all of the preferred features described herein. Preferred embodiments shall now be described with reference to the attached drawings, in which
- **Fig. 1**: shows a schematic representation of an example of a method for detecting plant diseases in plant material;
- **Fig. 2**: shows a schematic representation of an example of a method for detecting plant diseases in plant material;
- **Fig. 3**: shows an example of an analysis assembly for detecting plant diseases in plant material;
- **Fig. 4**: shows an example of a spectral signal obtained from using near-infrared spectroscopy on sugar beet pieces;
- **Fig. 5**: shows a schematic representation of an example of a method for generating calibration data for the determination of diseases in plant material.

In the figures, elements with the same or comparable functions are indicated with the same reference signs.

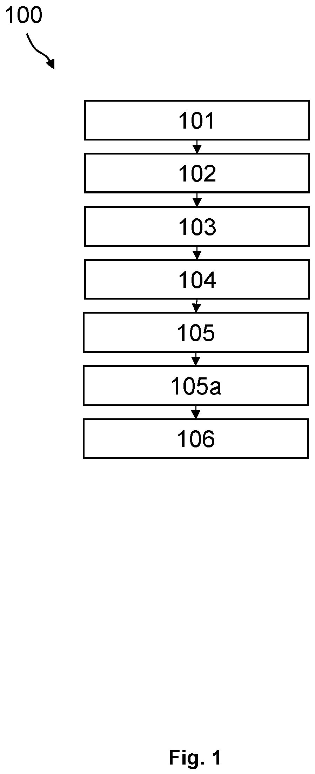

Fig. 1 shows a schematic representation of an example of a method 100 for detecting plant diseases in plant material. The method 100 comprises the steps described in the following. In a step 101, receiving plant material. In a step 102, emitting electromagnetic waves towards the plant material. In a step 103, receiving electromagnetic waves. In a step 104, converting the received electromagnetic waves into a spectral signal. In a step 105, determining plant diseases in the plant material. In a step 105a, generating calibration data, including taking a sample of plant material, preferably with and/or without plant diseases, calibration analysis of the plant material by evaluating plant diseases in the plant material, for example by visual scoring of a plant disease that is present in the plant material, spectroscopic analysis of the sample by, preferably continuously, emitting electromagnetic waves towards the sample, receiving electromagnetic waves, and converting the received electromagnetic waves into a spectral signal, comparing the results of the spectroscopic analysis with the results of the calibration analysis. In a step 106, comparing the spectral signal with calibration data and/or at least one reference value and dependent on the comparison determine presence and/or quantification and/or qualification of plant diseases in the plant material.

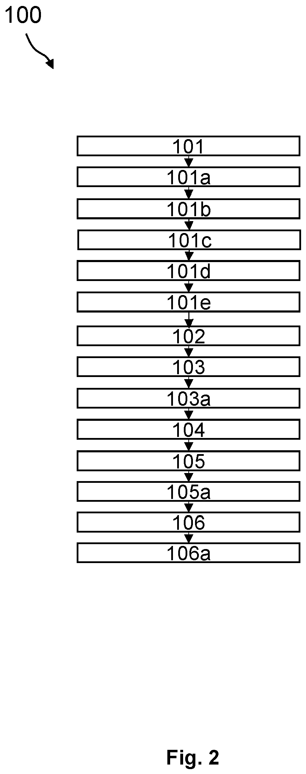

Fig. 2 shows a schematic representation of an example of a method 100 for detecting plant diseases in plant material. The method 100 comprises the steps described in Fig. 1 and the steps describes in following, wherein the preferred order of the steps can be seen in Fig. 2. In the following, only the steps that have not already been described in regard to Fig. 1 are described. In a step 101a, chopping and/or slicing the plant material into plant material pieces. In a step 101b, homogeneously distributing the plant material onto a transport device, preferably with a roller that is arranged above the transport device. In a step 101c, adjusting the plant material, in particular by adjusting the height of the plant material and/or by compressing the plant material and/or by smoothing the surface of the plant material, preferably with the roller. In a step 101d, arranging the, preferably homogeneously distributed, plant material onto a transport device. In a step 101e, conveying the plant material using the transport device, wherein preferably the plant material is conveyed towards a sensor, wherein the sensor is adapted to receive reflected electromagnetic waves. In a step 103a, continuously recording received reflected electromagnetic waves, and/or continuously converting the received electromagnetic waves into spectral signals. In a step 106a, introducing plant material into a breeding process, changing at least one breeding parameter, dependent on the determination of plant diseases in the plant material.

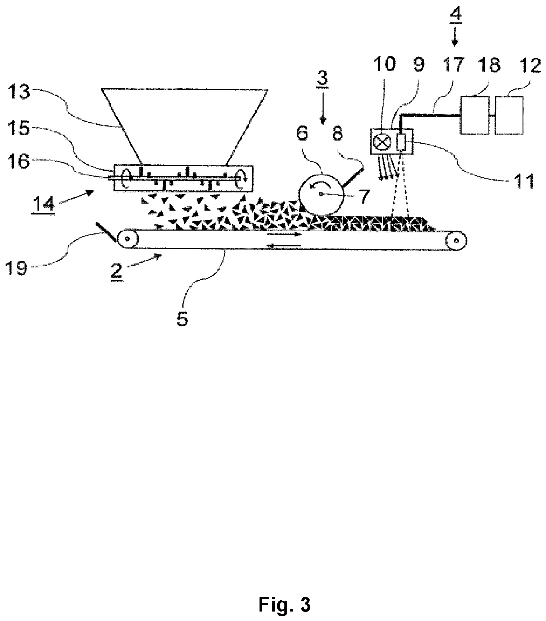

Fig. 3 shows an example of an analysis assembly 4 for detecting plant diseases in plant material. In the shown example, plant material, such as for example plant material from the species Beta vulgaris and/or Solanum tuberosum, in particular sugar beets and/or potatoes, are received in a receiving device 13. The plant material is cut and/or crumbled into plant material pieces using a cutting and/or crumbling device 14, comprising a rotating cutting part 16 in a housing 15. Subsequently, the plant material pieces fall onto a conveyor belt 5 of a transport device 2. As the plant material pieces contact the roller 6, they are spread on the conveyor belt 5 and are subject to a compressive force as a function of the distance between the roller 6 and the conveyor belt 5. The so-compressed plant material pieces have a smooth surface and a constant height. Scrapers 8,19 are provided on the roller 6 and the conveyor belt 5 and continuously clean the roller surface and belt during operation, thus avoiding the cross-mixing of two different plant material pieces for example from different samples. Moreover, a clumping or accumulation of plant material pieces on the conveyor belt 5 and the roller 6 can be ruled out, which could otherwise severely disturb the comparative homogenization of the sample flow. Directly downstream of the roller 6 a sensor head 9 is arranged with a light source 10 and a sensor 11 for detecting the electromagnetic waves from the smooth surface of the stream of plant material pieces in the wavelength range from 850 nm to 1650 nm. The sensor head 9 can be elevated at a fixed distance of 200 to 250 mm to the surface of the smooth flow of plant material pieces and can be pivoted as desired relative to the stream of plant material pieces. In this way, it is possible to sense and record the entire width of the stream of plant material pieces. The sensor 11 continuously records reflected electromagnetic waves and transmits it via an optical fiber 17 to a spectrometer 18, which converts the spectrally resolved radiation wavelengths into digitized, the spectral signals at regular intervals of 30 ms. Thus, during the flow-by of the stream of plant material pieces, several hundred such spectra are produced within a short period of time, which are filtered and averaged by a processor 12. By comparison with suitable calibration data, plant diseases can be determined in the plant material.

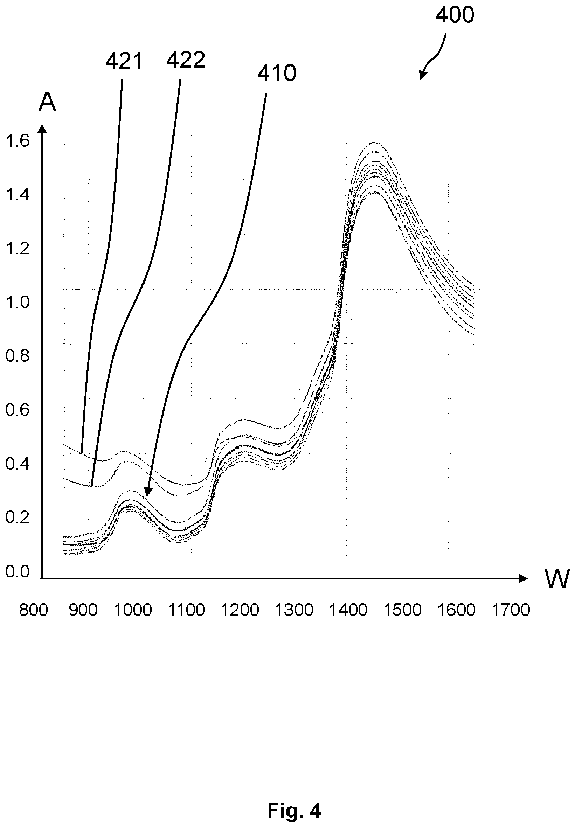

Fig. 4 shows an example of several spectral signals 400 obtained from using near-infrared spectroscopy on plant material, wherein in this example the plant material consists of sugar beet pieces. To generate spectral signals 400, first, electromagnetic waves are emitted towards sugar beet pieces that are arranged on a transport device by using an electromagnetic wave source. Then, reflected electromagnetic waves are received by using a sensor, wherein the sensor is arranged for detecting the reflected electromagnetic waves from the surface of the sugar beet pieces. Then, the received reflected electromagnetic waves are converted into a spectral signal using a near-infrared spectrometer. In the shown example, reflected electromagnetic waves in the wavelength range from 850 nm to 1650 nm are received. In the shown diagram, the absorbance A is plotted versus the wavelength W (in nm). During a stream of sugar beet pieces several of such spectral signals can be produced, which can be filtered and averaged by a processor. From such spectral signal 400 or several of such spectral signals, it is possible to detect plant diseases in sugar beet pieces.

The spectral signals 410 represent typical curves representing spectral signals of sugar beet pieces that are not infected with a plant disease. The spectral signals 421 and 422 show a deviation of the absorbance, in particular in the range of about 850 nm to 1300 nm. In this region, the absorbance is much lower for the spectral signals 421 and 422 when compared to the spectral signals 410. The spectral signals 421 and 422 represent typical curves representing spectral signals of sugar beet pieces that are infected with the plant disease "Rhizoctonia". From the deviation in the spectral signals obtained from healthy sugar beets and infected sugar beets, it is possible to automatically and reliably detect if plant diseases are present and if this is the case which plant diseases are present. For different plant diseases the deviations in the spectral signals caused by these plant diseases can be differently. This allows for distinguishing which plant disease or which plant diseases are present in the plant material from the information of the spectral signals.

For example, determining the plant disease "Rhizoctonia" can be achieved with an approach based on the slope of the spectral signal in the area of wavelengths between about 870 nm and 910 nm. Determining the slope in this region also allows a raw screening, wherein the screening is robust due to the nearly independence from the reference values and in particular from the absolute values of the absorbance. This is possible because of the significant influence of the plant disease "Rhizoctonia" on the slope in the area of wavelengths between about 870 nm and 910 nm.

Such spectral signals can be obtained for example by using an analysis assembly as shown in Fig. 3.

In particular, the method for determining plant diseases can be applied as described in the following example: Sugar beets with RHC (Rhizoctonia) and GTSC (girt scab) symptoms were collected and sorted by a visual scoring, wherein the visual scoring ranges from 1 to 9 with 1 for lowest and 9 for highest severity of visually detectable plant diseases. Each of these classes contained about 10-20 sugar beets. In the laboratory, first whole sugar beets were measured and then the same sugar beets were chopped using a chopping device. Then spectral signals obtained with NIRS were created and evaluated. A calibration was performed by multivariate regression methods between spectral signals and the rating done by visual scoring. The NIR spectral signal obtained from analysis on chopped sugar beets showed a better match with visual scoring than a NIR spectral signal obtained from non-chopped whole sugar beets. The calibration with normal filtering and only one component in the function, showed good results for GTSC. For RHC, the calibration showed good results with low filtering. This can be seen in the following table, were the correlation is indicated by R² and for most of the tests lies above 95. R² is the coefficient of correlation. Different filtering and different count of components were conducted.

| | **Filtering** | **component** | **R²** |
|---|---|---|---|
| **RHC** | low | 1 | 95.32 |
| **RHC** | normal | 5 | 97.73 |
| **RHC** | normal | 1 | 89.44 |
| **GTSC** | low | 1 | 96.90 |
| **GTSC** | normal | 1 | 96.36 |

The classical PLS approach works and allows a raw screening (low, medium and high infection). Also, an approach based on the slope of the spectra between 870 nm and 910 nm approach allows a raw screening and is robust due to the nearly independence from reference values. This is possible because of the significant influence of Rhizoctonia in this lower wavelength range.

The method can be applied for numerous plant diseases such as for example the following listed and described plant diseases that can occur in sugar beets:
1. RHIZOMANIA/ Beet necrotic yellow vein virus (BNYVV, Benyvirus), the causal agent of sugar beet rhizomania (Tamada & Baba, 1973). Rhizomania ('root madness or beardness') is characterized by the extensive proliferation of lateral rootlets along the main taproot, necrosis of the vascular bundle and severe stunting of the plant. BNYVV virions infect the sugar beet root system. In root sections a reddish-brown discoloration of the central stele can occur along with tumour-like symptoms from where the root proliferation appears. Severe infection can result in a high reduction of yield, sugar content and purity.
2. Beet soil-borne/Beet soil-borne virus (BSBV) causes no obvious symptoms on sugar beet. Heavily infected sugar beet plants can exhibit browning and necrosis of vascular bundles. Beet soil-borne mosaic virus (BSBMV) is a member of the genus Benyvirus. The roots of BSBMV-infected sugar beets are generally asymptomatic.
3. VIRUS YELLOWS/Currently, the sugar beet 'mild' yellowing viruses are classified within the family Luteoviridae of which there are three genera: Luteovirus, Polerovirus and Enamovirus. Within the Polerovirus genus there are three key beet-infecting species, Beet mild yellowing virus (BMYV), Beet western yellows virus-USA (BWYV) and Beet chlorosis virus (BChV). Initial symptoms of beet polerovirus-infected sugar beet are diffuse chlorotic areas on fully expanded leaves, and these areas eventually expand and merge.
4. Aphanomyces root rot/Abundant lateral roots are formed, which quickly become black and shrivelled. Root lesions start out yellow-brown and appear water-soaked, later becoming dark brown to black. The fungus may invade the lower portion of the taproot, inducing a tip rot.
5. Rhizoctonia root and crown rot/Roots show varied degrees of a dark brown to blackish rot, often beginning at the crown and extending down the taproot. Deep cankers or fissures are common in the crown area and on the side of affected roots, and brownish fungal hyphae may be visible within such cavities.
6. Violet root rot/Roots of affected plants exhibit purplish areas and a felt-like, reddish-purple mycelial growth that advances over the root surface from the tip to the crown, causing much soil adherence to diseased roots.
7. Phymatotrichum root rot/The fungus spreads over the root surface as a thin, felt-like layer of yellowish mycelium. Eventually, affected roots develop a rather superficial, yellow to tan rot.
8. Phytophthora root rot/Blackish spots appear toward the base of roots and a wet rot eventually spreads upward on the taproot. Rotted tissue is brown, with a blackish margin between healthy and diseased areas.
9. Pythium root rot/Grey to brown lesions appear on taproots, turning roots dark and spongy.
10. Phoma root rot/Dark brown, depressed lesions on the root surface near the crown are the first signs of this rot caused by Phoma betae. A soft watery rot develops beneath these lesions and spreads into neighbouring areas of the root. The rotting tissue is dark brown to black, subsequently darkening further and becoming dry and shrunken.
11. Beet vascular necrosis and rot/Root symptoms vary from soft to dry rot, and vascular bundles become necrotic. When the root is cut to expose the necrotic vascular bundles, surrounding areas turn pink or reddish within 20-30 seconds.
12. Crown gall/A tumorous outgrowth develops on the side of the root sometimes growing even larger than the root itself. The gall or galls tend to be covered by a corky or warty surface and are attached to the root by a relatively small neck of tissue.
13. Scab/Brown, round or oval, corky or scaly wart-like growths are found scattered over the root surface.
14. Low sugar syndrome (Basses richesses)/Affected plants may be scattered throughout the crop or occur at high density and their sugar content is reduced by 2-4 %.
15. Insects can as well directly or indirectly influence the quality of sugar beets.

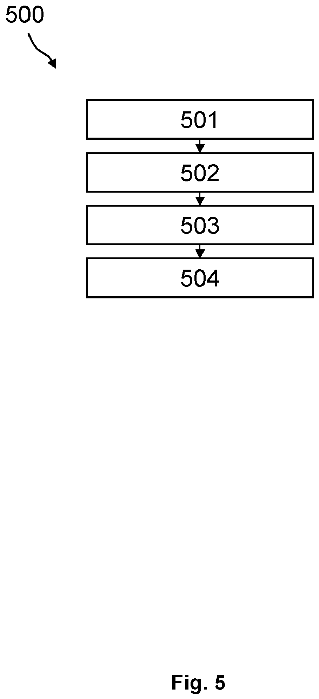

Fig. 5 shows a schematic representation of an example of a method for generating calibration data for the determination of diseases in plant material. The method 500 comprises the steps described in the following. In a step 501, taking a sample of plant material. In a step 502, calibration analysis of the plant material by evaluating plant diseases in the plant material, for example by visual scoring of a plant disease that is present in the plant material. In a step 503, spectroscopic analysis of the sample by, preferably continuously, emitting electromagnetic waves towards the sample, receiving electromagnetic waves, and converting the received electromagnetic waves into a spectral signal. In a step 504, comparing the results of the spectroscopic analysis with the results of the calibration analysis.

## Claims

1. A method (100) for detecting plant diseases in plant material and/or physiological properties influenceable by environmental stress in plant material, the method comprising
- receiving (101) plant material,
- emitting (102) electromagnetic waves towards the plant material,
- receiving (103) electromagnetic waves,
- converting (104) the received electromagnetic waves into a spectral signal (400), and preferably processing the spectral signal,
- determining (105) plant diseases in the plant material and/or physiological properties, in particular metabolites, influenceable by environmental stress in the plant material.

2. The method according to the preceding claim, comprising
- generating (105a) calibration data, including
∘ taking a sample of plant material, preferably with and/or without plant diseases and/or with and/or without physiological properties influenceable by environmental stress, and preferably moving the sample along a sensor that is adapted to receive electromagnetic waves,
∘ calibration analysis of the plant material by evaluating plant diseases in the plant material, for example by visual scoring of a plant disease and/or by analysis of metabolites and/or components present in the plant material,
∘ spectroscopic analysis of the sample by, preferably continuously, emitting electromagnetic waves towards the sample, receiving electromagnetic waves, and converting the received electromagnetic waves into a spectral signal,
∘ comparing the results of the spectroscopic analysis with the results of the calibration analysis;
and/or
- comparing the spectral signal with calibration data and/or at least one reference value and dependent on the comparison determine presence and/or quantification and/or qualification of plant diseases and/or physiological properties influenceable by environmental stress in the plant material.

3. The method according to at least one of the preceding claims,
wherein generating calibration data and/or generating the at least one reference value comprises conducting a plurality of spectroscopy analyses, in particular comprising generating a plurality of reference spectral signals, wherein preferably the plurality of reference spectral signals is converted from electromagnetic waves reflected from and/or transmitted through plant material without plant diseases and/or plant material with plant diseases, or at least a part thereof, and/or
wherein the calibration data is derived using multiple and/or multivariate and/or linear regression analysis, and/or
wherein the calibration data is derived from multivariate analysis including spectral preprocessing using principle component analysis (PCA), and/or multiple linear regression (MLR), and/or partial least squares (PLS) regression, and/or classification learning methods, and/ or clustering learning methods and/or machine learning, in particular using neuronal networks, and/or
wherein the spectral signal is classified into a classification of infestation classes and/or different plant diseases and/or metabolites.

4. The method according to at least one of the preceding claims,
wherein determining plant diseases and/or physiological properties influenceable by environmental stress in the plant material comprises processing the spectral signal for determining plant diseases and/or physiological properties influenceable by environmental stress in the plant material, and/or wherein processing the spectral signal and/or generating calibration data comprises one or several of the following steps:
- preprocessing the spectral signal for correcting and/or eliminating overlaying effects, wherein preferably preprocessing is conducted using Standard Normal Variate (SNV) and/or multiplicative scatter correction (MSC), and/or first derivatives, and/or second derivatives, and/or smoothing, and/or combination of Derivative and SNV, wherein preferably preprocessing is conducted before multiple and/or multivariate and/or linear regression analysis is carried out, and/or
- and/or Classification as a learning method that classifies data into one of numerous already defined definite classes. It requires pre-allocation of training data in order to classify unknown data, thus classification is a so-called supervised learning method. Relevant algorithms for supervised learning are K-nearest Neighbor (kNN), Decision Trees/Random Forest, Support Vector Machines (SVM), Discriminant Analysis (PLS-DA), Soft independent modelling by class analogy (SIMCA), Naive Bayes (NB) as well as classifiers based on neural network and machine learning and/or
- Clustering as an unsupervised technique and suited to explore unknown data. Clustering algorithms map or group the input data into clusters according to similarities between them. Such grouping occurs by density-based, hierarchical-based, partitioning or grid-based methods but can also be achieved by machine learning or deep learning approaches. Common algorithms are K-means, mean shift, Gaussian Mixture, hidden Markov model (HMM) and Density-Based Spatial Clustering of Applications with Noise (DBSCAN).
- removing spectral signals that are not converted from electromagnetic waves that are reflected from or emitted through the plant material, preferably by differentiating the spectral signals using classification and/or filtering, in particular using mathematical filtering methods, and/or
- averaging spectral signals to one spectral signal.

5. The method according to at least one of the preceding claims, comprising
- detecting metabolites and/or components of the plant material, and/or
- determining plant diseases in the plant material.

6. The method according to at least one of the preceding claims, comprising
- chopping and/or slicing (101a) the plant material into plant material pieces, and/or
- homogeneously distributing (101b) the plant material onto a transport device, preferably with a roller that is arranged above the transport device, and/or
- adjusting (101c) the plant material, in particular by adjusting the height of the plant material and/or by compressing the plant material and/or by smoothing the surface of the plant material, preferably with the roller; and/or
- arranging (101d) the, preferably homogeneously distributed, plant material onto a transport device (2), and/or
- conveying (101e) the plant material using the transport device, wherein preferably the plant material is conveyed towards a sensor (11), wherein the sensor is adapted to receive electromagnetic waves; and/or
- continuously recording (103a) received electromagnetic waves, and/or
- continuously converting the received electromagnetic waves into spectral signals.

7. The method according to at least one of the preceding claims,
wherein while emitting electromagnetic waves towards the plant material the plant material is arranged on the transport device (2) and/or conveyed using the transport device, and/or
wherein the wavelength of the electromagnetic waves lies in the ultraviolet (UV) spectrum and/or in the visible spectrum and/or in the infrared spectrum and/or in the microwave spectrum and/or in the terahertz-spectrum, wherein preferably the wavelength of the electromagnetic waves lies in the range of 10 nm to 3000 µm, and/or,
wherein the electromagnetic waves are received from at least one spectrometer and/or at least one spectrophotometer.

8. The method according to at least one of the preceding claims,
wherein chopping the plant material into plant material pieces is conducted using a chopping device (14), wherein the chopping device is configured to cut and/or crumble the plant material into substantially equal sized plant material pieces, and/or
wherein slicing the plant material into plant material pieces is conducted using a slicing device, wherein the slicing device is configured to cut and/or slice the plant material into plant material pieces that are preferably formed as thin elongated strips and/or slices.

9. The method according to at least one of the preceding claims,
wherein the species of the plant material is a root crop and/or a tuber crop, in particular Beta vulgaris and/or Solanum tuberosum, or at least a part thereof, and/or
wherein the plant material comprises or consists of sugar beets, and/or
wherein the plant material is introduced to a production process for processing the plant material in a processing industry, and/or
wherein the plant material is introduced to a breeding process.

10. The method according to at least one of the preceding claims, comprising
- introducing (106a) plant material into a breeding process, and/or
- changing at least one breeding parameter, dependent on the determination of plant diseases in the plant material and/or physiological properties influenceable by environmental stress in the plant material.

11. The method according to at least one of the preceding claims, comprising
- producing a product, preferably raw juice and/or sugar and/or starch, from the plant material,
- changing at least one sugar production parameter, in particular extraction parameters, and/or dosages of additives, and/or electric field pulses, and/or pulse numbers, and/or process temperature, and/or conveying speed and/or duration of plant material pieces in reactor and/or at least one drying process parameter, dependent on the determination of plant diseases and/or physiological properties changed by environmental stress in the plant material.

12. A method (500) for generating calibration data for the determination of diseases in plant material and/or physiological properties influenceable by environmental stress in plant material, comprising
- taking (501) a sample of plant material, preferably with and/or without plant diseases and/or with and/or without and/or physiological properties influenceable by environmental stress, and preferably moving the sample along a sensor that is adapted to receive electromagnetic waves,
- calibration analysis (502) of the plant material by evaluating plant diseases and/or physiological properties, in particular metabolites, influenceable by environmental stress in the plant material, for example by visual scoring and/or analysis of components and/or metabolism of a plant disease and/or physiological properties, in particular metabolites, influenceable by environmental stress present in the plant material,
- spectroscopic analysis (503) of the sample by, preferably continuously, emitting electromagnetic waves towards the sample, receiving electromagnetic waves, and converting the received electromagnetic waves into a spectral signal,
- comparing (504) the results of the spectroscopic analysis with the results of the calibration analysis.

13. An analysis assembly (4) for detecting plant diseases in plant material and/or physiological properties influenceable by environmental stress in plant material,
- wherein the analysis assembly is arranged to emit electromagnetic waves towards plant material pieces,
- wherein the analysis assembly is arranged to receive reflected and/or emitted electromagnetic waves,
- wherein the analysis assembly is arranged to convert the received reflected electromagnetic waves into a spectral signal (400),
- wherein the analysis assembly is processing the spectral signal for determining plant diseases and/or physiological properties, in particular metabolites, influenceable by environmental stress in the plant material.

14. An arrangement for detecting plant diseases in plant material and/or physiological properties influenceable by environmental stress in plant material, the system comprising
- a receiving section for receiving plant material,
- a chopping device (14) for chopping the plant material into plant material pieces and/or a slicing device for slicing the plant material into plant material pieces,
- an analysis assembly (4) according to claim 13.

15. A control unit for controlling an analysis assembly (4) according to claim 13 and/or for receiving data from an analysis assembly according to claim 13 and controlling at least one production parameter, in particular extraction parameters and/or dosages of additives, and/or electric field pulses and/or pulse numbers and/or process temperature and/or conveying speed and/or duration of plant material pieces in a reactor and/or at least one drying process parameter dependent on the determination of plant diseases in the plant material and/or physiological properties influenceable by environmental stress in the plant material.
